# EUROPEAN PATENT APPLICATION

(11) **EP 3 345 606 A1**
(43) Date of publication of application: **11.07.2018**
(21) Application number: 18157821.2
(22) Date of filing: 01.11.2013
(51) Int. Cl.: A61K 31/525, A61K 35/66, A61K 35/741, A61K 31/675, A61K 31/198, A23L 33/135, A23L 33/15, A23L 33/21

(54) **METHODS AND COMPOSITIONS FOR STIMULATING BENEFICIAL BACTERIA IN THE GASTROINTESTINAL TRACT**

(30) Priority: 01.11.2012 EP 12190948; 21.11.2012 US 201261728811 P
(62) Divisional of application: 13795881.5
(71) Applicant: RIJKSUNIVERSITEIT GRONINGEN, 9712 CP Groningen (NL); Academisch Ziekenhuis Groningen, 9713 GZ Groningen (NL)
(72) Inventor: Harmsen, Hermanus Josef Martinus, 9700 RB Groningen (NL); Khan, Muhammad Tanweer, 9700 RB Groningen (NL); Sadaghian Sadabad, Mehdi, 9700 RB Groningen (NL); Van Dijl, Jan Maarten, 9700 RB Groningen (NL)
(74) Representative: V.O.

(57) **Abstract**

The invention relates to supporting the growth or maintenance of oxygen-sensitive bacteria in the gastrointestinal tract of an animal. Provided is riboflavin, riboflavin phosphate or a salt thereof, for use in a method for the selective stimulation of *Faecalibacterium prausnitzii* in the gastrointestinal tract in an animal. Also provided is riboflavin for use in a method for preventing, treating or reducing the symptoms associated with an inflammatory gastrointestinal disorder, the method comprising administering to a human subject in need thereof an amount of riboflavin effective to maintain, support or stimulate the growth of F. prausnitzii in the gastrointestinal tract.

## Description

The present invention relates to methods and compositions to support the growth or maintenance of oxygen-sensitive bacteria in the gastrointestinal tract of an animal, preferably a mammal. Particularly, the invention relates to means and methods for selectively enhancing the growth of beneficial anaerobic butyrate-producing bacteria, such as *Faecaliumbacterium prausnitzii.*

Probiotics, also called beneficial bacteria, can provide beneficial effects to the hosts such as maintaining a normal host gastrointestinal microbiota and increasing resistance against pathogenic bacteria. Probiotic formulations have been used as a dietary supplement for many years. So far, many different probiotic strains and combinations thereof exist, but nearly all of them employ relatively-oxygen tolerant strains for instance *Bifidobacterium* sp., *Lactobacillus* sp. and *Saccharomyces* sp. Recent research in gut microbiology explores new horizons for probiotic applications, such as anti-inflammatory treatments and treatment of Crohn's disease, and may promote colonization resistance against pathogens. Such bacteria typically utilize a variety of carbohydrates and produce butyrate as the major fermentative end product. Butyrate is well known for its role in promoting and maintaining gut health. For example, *Faecaliumbacterium prausnitzii* was found to exhibit anti-inflammatory effects on cellular and TNBS colitis models, partly due to secreted metabolites able to block NF-κB activation and IL-8 production. *F. prausnitzii* is one of the most abundant human colon bacteria with numbers ranging from 5-20% of the total microbiota in stools of healthy individuals. It was found that a reduction of *F*. *prausnitzii* is associated with a higher risk of postoperative recurrence of ileal Crohn's Disease (Sokol et al. PNAS, 2008, Vol.105, No.43, 16731-16736). The current idea is that counterbalancing the *F. prausnitzii* dysbiosis is a promising strategy in CD treatment by preventing reoccurrence of exacerbations.

Particular ingredients are known to support the growth or maintenance of beneficial bacteria so as to modify the gastrointestinal microbial community in a beneficial manner. Such ingredients are called "prebiotics." Typical examples of known prebiotics are oligosaccharides, such as fructooligosaccharides and inulin. Synbiotics refer to nutritional supplements combining probiotics and prebiotics in a form of synergism Numerous prebiotic and synbiotic formulations are known in the art to maintain or stimulate the level of beneficial oxygen-tolerant bacteria in the gut. In contrast, very few studies have dealt with stimulating oxygen-sensitive bacteria, presumably because of the technical difficulties to keep these bacteria alive. For example, bacteria needed for the afore-mentioned anti-inflammatory effects are extremely sensitive to oxygen and cannot survive an exposure to ambient air for more than a few minutes. As a consequence, probiotic compositions containing *F. prausnitzii* have not been described thus far despite their promising therapeutic application.

Recognizing the therapeutic potential of beneficial anaerobic bacteria, the present inventors set out to identify new means and methods to enhance the population of butyrate-producing anaerobic bacteria in the gastrointestinal tract. In particular, they aimed at providing novel prebiotic and synbiotic formulations for selectively stimulating butyrate-producing anaerobic bacteria, preferably *F. prausnitzii.*

It was surprisingly found that orally administered riboflavin, also known as vitamin B2, is capable of increasing the absolute and relative number of concentration of *F*. *prausnitzii* in human volunteers. Butyrate production was also enhanced. Furthermore, a synbiotic formulation was developed to stabilize *F. prausnitzii* under aerobic conditions, thus allowing the use of this highly oxygen-sensitive organism as probiotic.

Accordingly, in one embodiment the invention provides the use of riboflavin, riboflavin phosphate or a physiologically acceptable salt thereof, for the manufacture of a food composition, pharmaceutical composition, food or dietary supplement, for the selective stimulation of *F. prausnitzii* in the animal gastrointestinal tract,. wherein riboflavin is used in an amount of 0.01 to 2 mg per kg body weight per day.

Riboflavin, also known as vitamin B2, is a micronutrient with a key role in maintaining health in humans and other mammals. It is the central component of the cofactors FAD and FMN, and is therefore required by all flavoproteins. As such, riboflavin is required for a wide variety of cellular processes. It plays a key role in energy metabolism, and for the metabolism of fats, ketone bodies, carbohydrates, and proteins. Riboflavin is found naturally in asparagus, popcorn, bananas, persimmons, okra, chard, cottage cheese, milk, yogurt, meat, eggs, fish, and green beans. Other sources specify cheese, leafy green vegetables, liver, kidneys, legumes, tomatoes, yeast, mushrooms, and almonds.

Riboflavin has been used in several clinical and therapeutic situations. For over 30 years, riboflavin supplements have been used as part of the phototherapy treatment of neonatal jaundice. The light used to irradiate the infants breaks down not only bilirubin, the toxin causing the jaundice, but also the naturally occurring riboflavin within the infant's blood, so extra supplementation is necessary. A high dose riboflavin appears to be useful alone or along with beta-blockers in the prevention of migraine. A dose of 400 mg daily has been used effectively in the prophylaxis of migraines, especially in combination with a daily supplement of magnesium citrate 500 mg and, in some cases, a supplement of coenzyme Q10. Riboflavin in combination with UV light has been shown to be effective in reducing the ability of harmful pathogens found in blood products to cause disease. Recently, riboflavin has been used in a new treatment to slow or stop the progression of the corneal disorder keratoconus. Prior to the invention, a role for riboflavin as prebiotic for beneficial gut bacteria has never been disclosed.

Riboflavin can be used as such, or it can be incorporated in any desirable formulation suitable for oral intake. The formulation can be liquid or solid. Preferably, it is a food product, pharmaceutical composition, food or dietary supplement. For example, the food product is a milk or yoghurt drink. In a preferred aspect, riboflavin is comprised in a coated capsule, allowing for delivery of riboflavin in the colon. In some embodiments, e.g. to enhance incorporation of riboflavin into a liquid product, the more soluble form riboflavin-5'-phosphate (E101a) or the monosodium salt of the 5'-monophosphate ester of riboflavin (E106), may be used.

It was found that selective stimulation of *Faecalibacterium prausnitzii* by riboflavin can be achieved using an amount of at least 0.01 to 2 mg per kg body weight per day to ensure that enough riboflavin reaches the large intestine/colon and not all is absorbed in the small intestine. The riboflavin dose found to be effective for the purpose of the present invention is about 50 times higher than the recommended daily intake (1.5 mg/day for adult men) and at least 10 times higher than the 6-10 mg/day for treating riboflavin deficiency. However, the dosage, frequency and duration of riboflavin supplementation for depend on various factors such as the individual's state of health and weight. They can be determined by the skilled person based on his general knowledge and experience. In one embodiment, riboflavin is used in an amount of 0.1 to 1000 mg per kg body weight, preferably 1-100 mg per kg body weight, per day. Riboflavin is preferably used for at least 3 consecutive days, preferably at least 7 days, more preferably at least 10 days. Very good results were obtained in human individuals after one oral dose of at least 50 mg, preferably at least 100 mg riboflavin per day for a period of 14 days or more. However, a lower or higher frequency, dosage and/or total treatment period is also envisaged. Preferably, riboflavin is administered as a single daily dose.

A further aspect of the invention relates to the use of riboflavin, riboflavin phosphate or a physiologically acceptable salt thereof, as prebiotic for *F. prausnitzii.* This prebiotic use has important therapeutic applications. As used herein, prebiotic use refers to the use of riboflavin to stimulate the growth and/or activity of *F. prausnitzii* in the digestive system, preferably by selectively increasing the number and relative percentage of *F. prausnitzii* in the gut. The bacteria may be of endogenous and/or exogenous origin (e.g. upon intake of a symbiotic composition of the invention). The abundance of *F. prausnitzii* (and that of other bacteria) in the gastrointestinal tract can be readily determined by analysis of fecal samples using methods known in the art, in particular using molecular techniques such as FISH analysis, quantitative real time PCR or high-throughput 16S rRNA sequencing. Activity of *F*. *prausnitzii* can be determined by measuring beneficial fermentation products, preferably butyrate.

In yet another aspect, the invention provides a method for the selective stimulation of *F. prausnitzii* in the gastrointestinal tract in an animal, e.g. a mammal, in need thereof, comprising administering to the animal riboflavin, riboflavin phosphate or a physiologically acceptable salt thereof, in an amount effective to selectively stimulate the growth of *F. prausnitzii* in the gastrointestinal tract. The animal can be a human, pet or livestock. Thus, veterinary use of the present invention is also encompassed. For example, the invention also provides a method for enhancing *F*. *prausnitzii* numbers in pets or livestock. In a specific aspect, the livestock is poultry, e.g. a chicken. For example, the invention provides a method to maintain, support or stimulate the growth of *F. prausnitzii* in the gizzard of a bird. The gizzard, also referred to as the ventriculus, gastric mill, and gigerium, is an organ found in the digestive tract of some animals, including birds, reptiles, earthworms, some gastropods and some fish.

Preferably, the animal is a human subject. In one embodiment, the human subject is suffering from an inflammatory gastrointestinal disease, in particular Crohn's disease or a related colitis. Accordingly, also provided is a method for preventing, treating or reducing the symptoms associated with an inflammatory gastrointestinal disorder, comprising administering to a subject in need thereof an amount of riboflavin effective to maintain, support or stimulate the growth of *F. prausnitzii* in the gastrointestinal tract. Individuals with exemplary inflammatory gastrointestinal disorders, who may benefit from increasing *F. prausnitzii* numbers in the GI tract by riboflavin, include patients with Crohn's disease, inflammatory bowel disease and ulcerative colitis. Also encompassed is the treatment of other diseases, conditions or disorders where patients benefit from restoring or increasing *F. prausnitzii* numbers in the GI tract.

Another aspect of the invention relates to a synbiotic composition comprising living butyrate-producing anaerobic bacteria mixed with riboflavin, riboflavin phosphate or a physiologically acceptable salt thereof, and cysteine. This mixture was found to give a surprisingly good protection of these bacteria against exposure to ambient air during manufacturing, storage and/or consumption. For example, it can withstand ambient air exposure for at least 24 h, and its stability in simulated gastrointestinal fluid for at least 2 h can be observed. Also, the synbiotic formulation according to the invention can be stable for at least 2 h when mixed with a food product, for instance milk or yoghurt drinks. Preferably, riboflavin is used. Herewith, the invention provides a stable and cost-effective symbiotic formulation, which is stable upon exposure to oxygen. Cystein replacement by other anti-oxidants, such as quercitin or ascorbic acid did not yield the desired result, thus indicating the surprisingly unique contribution of cystein.

The synbiotic combination according to the present invention is beneficial as a food supplement for re-introducing or for increasing the numbers of beneficial (i.e. food-grade non-pathogenic) butyrate-producing anaerobic bacteria, in the animal intestinal tract. Generally, the bacteria will be administered in a therapeutically effective dose and/or in a prophylactically effective dose. If the bacteria are present in a viable form, it is theoretically effective in any concentration considering the fact that these bacteria can colonize the gut and multiply.

For the composition of the present invention it is generally preferred that a daily dose of the composition comprises between 10exp4 and 10exp12 cfu (colony forming units) of butyrate-producing anaerobic bacteria. A particular suitable daily dose is from 10exp6 to 10exp10 cfu. The composition of the present invention may comprise between 10exp2 and 10exp10 cfu of butyrate-producing anaerobic bacteria, preferably 10exp6 to 10exp9 colony forming units, more preferably from 10exp6 to 10exp8 cfu of bacteria, per gram dry weight of the composition.
In one embodiment, the butyrate-producing anaerobic bacterium is a member of the phylum Firmicutes, preferably of the Clostridium leptum phylogenetic group. In a preferred aspect, the symbiotic composition comprises *F. prausnitzii.* The composition of the present invention may comprise between 10exp2 and 10exp10 cfu of *F. prausnitzii*, preferably 10exp6 to 10exp9 colony forming units, more preferably from 10exp6 to 10exp8 cfu of *F. prausnitzii*, per gram dry weight of the composition. *F*. *prausnitzii* strain VPI C13-20-A, ATCC number 29739, is available from LGC/ATCC American Type Culture Collection, LGC Standards, Wesel, Germany. *F. prausnitzii* strain A2-165; DSM number 17677 is available from DSMZ-German Collection of Microorganisms and Cell Cultures, Braunschweig, Germany

Mixtures of two or more distinct bacteria that produce butyrate may be present, for example bacteria selected from the group of Ruminococci, Bifidobacteria and Lachnospiraceae. In one embodiment, additional butyrate-producers are selected from those belonging to the genera Roseburia or Anaerostipes, or the species *Eubacterium hallii.*

The riboflavin, riboflavin phosphate or a physiologically acceptable salt thereof, is present in an amount of at least 0.05%, preferably at least 1%, more preferably at least 2% based on the total dry weight of the composition. For example, it may contain 0.05 to 10%, preferably 1-10%, like 2-10%, or 0.05-0.25% based on the total dry weight of the composition. Cystein is preferably present in an amount of at least 0.05% based on the total dry weight of the composition. A cysteine content up to 2% is suitably used. For example, the composition may contain 0.1-1.5%, 0.5-1% or 0.05-0.2% cystein based on the total dry weight of the composition.
Generally speaking, a product with cysteine and riboflavin is compact and well protected. However, it may be technically difficult to formulate. If bulking agents (e.g. corn starch or wheat bran) are added, it will increase the bulk volume and make it easy for oxygen to penetrate and it will attract moisture. Coating the granules containing bacteria and anti-oxidants with inulin before the bulking agents are added is essential to increase the product stability.

Hence, it is preferred that the composition comprises inulin or inulin-type fructooligosaccharide (FOS). Inulins are polymers composed mainly of fructose units, and typically have a terminal glucose. The fructose units in inulins are joined by a β(2→1) glycosidic bond. In general, plant inulins contain between 20 and several thousand fructose units. Inulin-type FOS is produced by degradation of inulin, or polyfructose, a polymer of D-fructose residues linked by β(2→1) bonds with a terminal α(1→2) linked D-glucose. The degree of polymerization of inulin ranges from 10 to 60. Inulin can be degraded enzymatically or chemically to a mixture of oligosaccharides with the general structure Glu-(Fru)ₙ (GFₙ) and Fruₘ (Fₘ), with n and m ranging from 1 to 7. Inulin-type FOS is suitably used at about 2%-10% on the basis of weight percent.

Very good survival rates after exposure of the bacteria to ambient air were obtained when riboflavin and cystein were supplemented with inulin-type oligosacchararides, resistant starch and wheat bran. Resistant starch (RS) refers to starch and starch degradation products that escape digestion in the small intestine of healthy individuals. The symbiotic formulation preferably comprises resistant starch 40% - 65% on a dry weight basis. Corn starch is preferred. Wheat bran is a common fiber source of our diet and considered a rich source of insoluble non-starch polysaccharides and vitamins. Preferably, in the current symbiotic formulation wheat bran makes up 20% - 40% of the composition on a dry weight basis. Wheat bran may be replaced by buckwheat bran, a gluten free alternative bran with similar properties, e.g. for the use in celiac disease patients.
Accordingly, the invention also provides a method for protecting butyrate-producing anaerobic bacteria, preferably *F*. *prausnitzii*, against detrimental effects of exposure to ambient air, comprising formulating the bacteria into a composition comprising riboflavin and cystein, preferably further comprising inulin, resistant starch and (buck)wheat bran. As used herein, "protection against detrimental effects of exposure to ambient air" refers to maintaining at least 50%, preferably at least 70%, more preferably at least 90% of the initial number of CFU upon 24 h storage.
The inventors observed that a foamy material which is difficult to handle was obtained when the faecalibacteria were formulated with cysteine, riboflavin and inulin. Therefore, they tested the possibility to use corn starch and wheat bran as bulking agents. Notably, these two compounds had no protective effects on the faecalibacteria, neither by themselves nor in combination with inulin (Table 1 and data not shown). However, close to maximum faecalibacterial survival (∼60%) was observed when corn starch and wheat bran were combined with inulin, riboflavin and cysteine. This finding is important, because the mixture yields hard and compact granules that have a considerable bulk volume. Thus, riboflavin and cysteine provide the protective effect whereas inulin is a stabiliser. The starch and wheat bran are bulking agents to allow for facile fabrication of the formulation. Accordingly, also provided is a method for protecting *Faecalibacterium prausnitzii* bacteria against detrimental effects of exposure to ambient air, comprising formulating *Faecalibacterium prausnitzii* into a composition comprising (i) riboflavin, riboflavin phosphate or a physiologically acceptable salt thereof, (ii) cystein, (iii) inulin, (iv) corn starch and (v) wheat bran, and wherein said formulating comprises providing granules containing the bacteria, the riboflavin and the cystein and coating said granules with inulin before adding the corn starch and wheat bran.

Also of interest is the addition of one or more further cryoprotectants, such as trehalose. A combination of inulin and trehalose is preferred.

Of course, the composition may contain further useful ingredients, including further prebiotics and/or probiotics. Useful probiotic bacteria are preferably selected from the group consisting of lactic acid bacteria, bifidobacteria or mixtures thereof. Probiotic bacteria may be any lactic acid bacteria or bifidobacteria with established probiotic characteristics. For example, they may be also capable of promoting the development of a bifidogenic intestinal microbiota. Suitable additional probiotics may be selected from the group consisting of Bifidobacterium, Lactobacillus, Streptococcus and Saccharomyces or mixtures thereof, in particular selected from the group consisting of Bifidobacterium longum, Bifidobacterium lactis, Lactobacillus acidophilus, Lactobacillus rhamnosus, Lactobacillus paracasei, Lactobacillus johnsonii, Lactobacillus plantarum, Lactobacillus salivarius, Enterococcus faecium, Saccharomyces boulardii and Lactobacillus reuteri or mixtures thereof, preferably selected from the group consisting of Lactobacillus johnsonii (NCC533; CNCM 1-1225), Bifidobacterium longum (NCC490; CNCM 1-2170), Bifidobacterium longum (NCC2705; CNCM 1-2618), Bifidobacterium lactis (2818; CNCM 1-3446), Lactobacillus paracasei (NCC2461; CNCM 1-2116), Lactobacillus rhamnosus GG (ATCC53103), Lactobacillus rhamnosus (NCC4007; CGMCC 1.3724), Enterococcus faecium SF 68 (NCIMB10415), and mixtures thereof. In one embodiment, the further probiotic comprises food-grade bacteria which may be recombinant non-pathogenic food-grade bacteria serving as delivery vehicles of an anti-inflammatory molecule. See for example WO2011/086172 and references cited therein. Useful growth substrates include cellobiose and lactulose. The formulation may contain fillers and extenders, such as maltodextrin or pullulan.

Also provided is a method for preparing a symbiotic composition of the invention. The afore-mentioned described components may be blended by conventional wet granulation and freeze-dried into the powdered form. The composition of the present invention may be provided in powder form having a water activity smaller than 0.2, for example in the range of 0.19-0.05, preferably smaller than 0.15. The composition can be a shelf-stable powder. The low water activity provides this shelf stability and ensures that the probiotic micro-organism will remain viable even after long storage times. Water activity or A_{w} is a measurement of the energy status of the water in a system. It is defined as the vapour pressure of water divided by that of pure water at the same temperature; therefore, pure distilled water has a water activity of exactly one. The powder obtained can be compressed into tablets, filled in capsules or in tetra pack sachets for oral administration.

A further embodiment of the invention relates to the use of a symbiotic composition, for example as food supplement, dietary supplement or therapeutic agent. In one embodiment, it may be used in a method for increasing or restoring *F*. *prausnitzii* numbers in the mammalian gastrointestinal tract, optionally in combination with the use of additional riboflavin as prebiotic to selectively stimulate growth of *F*. *prausnitzii.* The synbiotic formulation may be administrated orally at a dosage rate ranging from 100 mg to 1000 mg per day.

### LEGENDS TO THE FIGURES

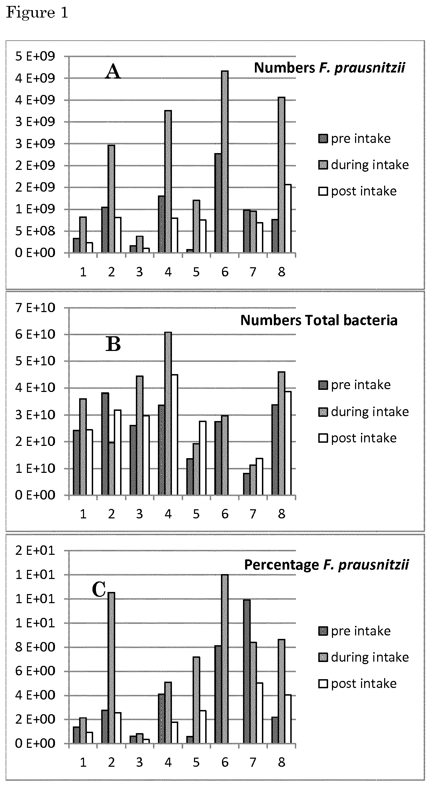

Figure 1: The number and percentages of *F*. *prausnitzii* and total bacteria in fecal samples of 8 volunteers during an intervention trial with 100 mg/day of riboflavin for two weeks. (Top panel) number of *F*. *prausnitzii* per gram: pre intake (average of two samples), during intake (average of two samples) and post intake (one sample); (Middle panel) total bacteria; (Bottom panel) percentage of *F*. *prausnitzii* of the total bacteria.

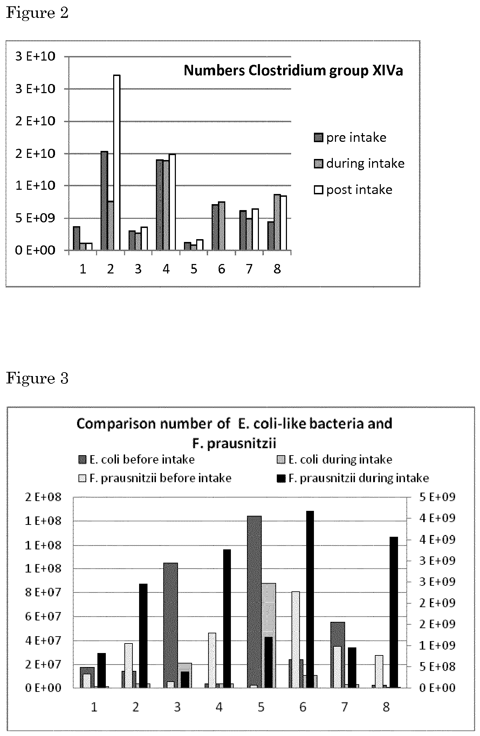

Figure 2: The number of Clostridium group XIVa bacteria in fecal samples of 8 volunteers pre, during and post intervention with 100 mg/day of riboflavin for two weeks.
Figure 3: Comparison of the number of *E. coli*-like bacteria and *F*. *prausnitzii* (cells/g) in fecal samples of 8 volunteers pre, during and post intervention with 100 mg/day of riboflavin for two weeks. In this comparison *F*. *prausnitzii* numbers (right axis) are plotted in front of the *E. coli*-like numbers (left axis). Note the inverse relation.

### EXPERIMENTAL SECTION

### Example 1: Riboflavin intervention trial.

A human intervention study was performed to investigate the effect of riboflavin on the numbers of *Faecalibacterium prausnitzii* in the gut. A group of 8 volunteers (body weight 60 to 90 kg) were asked to take 1 oral dose of 100 mg riboflavin supplement per day for 14 days.

Two samples were taken in a period of two weeks before the intake, as baseline samples, two samples (one per week) were taken during the intake and one sample a week after intake. The number of *F*. *prausnitzii* and that of other bacterial groups were determined by FISH with a specific probes as describe before (Harmsen et al. Appl Environ Microbiol. 2002 Jun; 68(6):2982-90). The numbers of faecalibacteria of the samples taken before and during the intake were averaged and all samples were plotted in Figure 1. The results show that in all volunteers tested, except for individual 7, the number of faecalibacteria increased during intake and decreased again when the intake ceases.

The number of *F*. *prausnitzii* also increased relatively to the other bacteria in the feces. The percentage *F*. *prausnitzii* increased in 7 out of 8 samples upon riboflavin intake. Other groups of bacteria, such as the butyrate producing *Clostridium* group XIVa did not increase (Fig. 2). The ratio faecalibacteria/Clostridium group XIVa, calculated from before and from during intake, increased in all cases, showing a relative increase of faecalibacteria. Also, the numbers of two potentially pathogenic bacteria were counted, such as *Enterobacteriaceae*, *E. coli*-like bacteria of which *E. coli* is the most abundant in the gut, and Enterococci. This last group was only detected in very low numbers that did not increase during the intervention. The number of *E. coli* showed much variation among the samples (Fig. 3). However, the number decreased or remained the same during riboflavin intake in all individuals tested. In addition, an inverse relation was detected between the numbers of faecalibacteria and *E. coli-*like bacteria (Fig 3, lower panel). When *E. coli* was high, the number of faecalibacteria was relatively low. This situation improved i.e. the ratio shifted towards faecalibacteria, upon administration of riboflavin.

### Example 2: Stability of F. prausnitzii synbiotic formulations after exposure to ambient air.

To produce a formulation with living faecalibacteria that are stable for at least 24 h under aerobic conditions, series of experiments were performed with different combinations of ingredients. Faecalibacteria were grown overnight in broth culture medium, centrifuged and the pellet was washed with anaerobic phosphate buffered saline (PBS) and centrifuged again. The pellets were mixed with PBS containing the ingredients as shown in Table 1. The mixtures were frozen at -20°C and subsequently lyophilized.

After lyophilizing, the formulations were exposed to air for the indicated time periods. The preparations were re-suspended in phosphate buffer and a 10 fold-dilution series was plated on anaerobic growth medium agar. The Colony-forming units were counted and compared with a non exposed t=0 time point.

**Table 1: Survival of F. prausnitzii in different formulations**

| **Combinations** | | | | | | **Survival percentage (+/- 5%)** | | |
|---|---|---|---|---|---|---|---|---|
| | | | | | | **Exposure to ambient air (h)** | | |
| | | | | | | **6** | **11** | **24** |
| **1** | Inu | | | | | nd | nd | 0 |
| **2** | | Rb | | | | nd | nd | 0 |
| **3** | | | Cys | | | nd | nd | 10 |
| **4** | Inu | Rb | | | | nd | nd | 0 |
| **5** | Inu | | Cys | | | nd | nd | 59 |
| **6** | | Rb | Cys | | | nd | nd | 60 |
| **7** | Inu | Rb | Cys | | | nd | nd | 70 |
| **8** | | | | Cs | | 0 | 0 | 0 |
| **9** | | | | Cs | WB | 0 | 0 | 0 |
| **10** | Inu | | | Cs | WB | 6 | 0 | 0 |
| **11** | Inu | Rb | Cys | Cs | WB | 93 | 100 | 57 |
| **12** | Inu | Rb | | Cs | WB | 75 | 3 | 0 |
| **13** | | Rb | Cys | Cs | WB | nd | 45 | 0 |
| **14** | Inu | Rb | Cys | Cs | | nd | 40 | 22 |
| **15** | | | Cys | Cs | | nd | 53 | 0 |
| **16** | Inu | | | | WB | nd | nd | 0 |

| | | | | | | | | |
|---|---|---|---|---|---|---|---|---|
| Inu, Inulin; Rb, Riboflavin; Cys, Cysteine; Cs, Corn starch; WB, Wheat bran; nd, not determined. | | | | | | | | |

Table 1 exemplifies that the stability of *F*. *prausnitzii* in the synbiotic formulations depends on several factors:
- The presence of antioxidants cysteine and riboflavin that have reversible oxidation-reduction reactions. The protective effect is not observed for other anti-oxidants like quercitin or ascorbic acid. Since quercetin is irreversible oxidized and ascorbic acid is not stable when moisture is present, these do not have a protective effect.
- For a good protection from oxygen penetration by the anti-oxidant-redox mediators, either compactness of the granules or coating with a cryo-preservant such as inulin, is preferred. The addition of wheat bran or corn starch is only effective if inulin coating is performed. Inulin also attracts less moisture than wheat bran or corn starch.

### Example 3: Exemplary synbiotic compositions

### Step-1

### Cultivation of bacteria

The *F*. *prausnitii* strain A2-165 (DSM 17677) was revived from glycerol stocks (-80° C) by inoculation 10µl of samples on yeast extract, casitone, fatty acid and glucose (YCFAG) agar medium. The cultures were cultivated and routinely maintained on YCFAG medium in an anaerobic tent with H₂ 10%, CO₂ 10% and N₂ 80% (v/v) gas mixture at 37° C. The starter culture was obtained by inoculating single colony of *F*. *prausnitzii* strain into 5ml of YCFAG broth and cultivated for 12h to 16h to an optical density (OD) at 600 nm (A600) of ∼0.8. The 1 ml of the starter culture was inoculated into 50 ml of the fresh YCFAG medium (2 % v/v inoculum). The culture was incubated for 12h-16h until it reached an OD_{A600} of ∼0.8± 0.2. Cells were harvested by centrifugation at 2700g at 17°C for 10 min. The pellet was washed once with sterile anaerobic saline solution (0.85 % NaCl and 0.05 % cysteine).

### Step-2

### Formulation procedure

### Combination 1: Synbiotic fibre mixture with inulin.

The bacterial pellet (containing 5 x 10⁸- 2 x 10⁹ bacteria) obtained according to step-1 was suspended in 0.4 ml solution containing 10% (w/v) inulin and 16.5 mM cysteine. After resuspending the pellet, 0.2 ml of the stock solution (16.5 mM) of riboflavin was added. After thorough mixing, 0.5 g of wheat bran and 0.9 g of corn starch was added and the resulting slurry was wet granulated. The wet granules were freeze at -20°C for at least 3h. After freezing, the granules were lyophilized for at least 3h.

### Combination 2: Synbiotic fibre mixture with trehalose

The bacterial pellet obtained according to step-1 was suspended in 0.4 ml solution containing 10% trehalose and 16.5 mM cysteine. After resuspending the pellet, 0.2 ml of the stock solution (16.5 mM) of riboflavin was added. After thorough mixing, 0.5 g of wheat bran and 0.9 g of corn starch was added and the resulting slurry was wet granulated. The wet granules were frozen at -20°C for at least 3h. After freezing, the granules were lyophilized for at least 3h.

### Combination 3: Synbiotic mixture with inulin or trehalose

The bacterial pellet obtained according to step-1 was suspended in 1.6 ml solution containing 10% inulin or trehalose and 16.5 mM cysteine, a solution that is purged with N₂ before addition of cysteine. After resuspending the pellet, 0.2 ml of the stock solution (16.5 mM) of riboflavin was added. After thorough mixing the resulting slurry was frozen at -20°C for at least 3h. After freezing, the granules were lyophilized for at least 3h.

**Combination 4: Probiotic mixture** The bacterial pellet obtained according to step-1 was suspended in 0.4 ml solution 16.5 mM cysteine. After resuspending the pellet, 0.2 ml of the stock solution (16.5 mM) of riboflavin was added. After thorough mixing the resulting slurry was frozen at -20°C for at least 3h. After freezing, the granules were lyophilized for at least 3h.

### Combination 5: Synbiotic fibre mixture with inulin and extra riboflavin.

The bacterial pellet (containing 5 x 10⁸- 2 x 10⁹ bacteria) obtained according to step-1 was suspended in 0.4 ml solution containing 10% (w/v) inulin and 16.5 mM cysteine. After resuspending the pellet, 0.2 ml of the stock solution (100 mM) of riboflavin was added. After thorough mixing, 0.5 g of wheat bran and 0.9 g of corn starch was added and the resulting slurry was wet granulated. The wet granules were freeze at -20°C for at least 3h. After freezing, the granules were lyophilized for at least 3h.

### Step-3

### Procedure for stability and viability assay

The lyophilized granules obtained according to step 2 were stored in air tight containers under ambient aerobic conditions. For stability and viability studies granules were exposed to the ambient air at defined period up to 24h. After aerobic exposures the granules were processed anaerobically under anaerobic tent. The granules were diluted and rehydrated in oxygen free phosphate buffered saline (pH 7.2) supplemented with 0.05% cysteine and ten-fold dilution series were plated on YCFAG agar medium. Colony forming units were determined by counting the colonies formed on the agar plates after 24h of incubations at 37°C.

## Claims

1. Riboflavin, riboflavin phosphate or a physiologically acceptable salt thereof for use in a method for the selective stimulation of *F*. *prausnitzii* in the gastrointestinal tract in an animal, comprising administering to the animal riboflavin, riboflavin phosphate or a physiologically acceptable salt thereof in an amount effective to selectively stimulate the growth of *F.prausnitzii* in the gastrointestinal tract.

2. Riboflavin, riboflavin phosphate or a physiologically acceptable salt thereof for use according to claim 1 wherein the animal is a human, pet or livestock.

3. Riboflavin for use in a method for preventing, treating or reducing the symptoms associated with an inflammatory gastrointestinal disorder, the method comprising administering to a human subject in need thereof an amount of riboflavin effective to maintain, support or stimulate the growth of *F.prausnitzii* in the gastrointestinal tract.

4. Riboflavin for use according to claim 3, wherein the inflammatory gastrointestinal disorders are inflammatory bowel diseases.

5. Riboflavin for use according to claim 4, wherein the inflammatory bowel diseases are Crohn's disease and ulcerative colitis.

6. Riboflavin, riboflavin phosphate or a physiologically acceptable salt thereof for use according to anyone of the preceding claims, wherein riboflavin is used in an amount of 0. 1 to 1000 mg per kg body weight, preferably 1-100 mg per kg body weight, per day.

7. Riboflavin, riboflavin phosphate or a physiologically acceptable salt thereof for use according to any one of the preceding claims, wherein riboflavin is used for at least 3 consecutive days, preferably at least 7 days, more preferably at least 10 days.
